# EUROPEAN PATENT APPLICATION

(11) **EP 3 124 042 A1**
(43) Date of publication of application: **01.02.2017**
(21) Application number: 15178653.0
(22) Date of filing: 28.07.2015
(51) Int. Cl.: A61K 39/00, C07K 16/10

(54) **IMMUNOGLOBULIN SINGLE VARIABLE DOMAIN ANTIBODY AGAINST RSV PREFUSION F PROTEIN**

(71) Applicant: VIB vzw, 9052 Gent (BE); Universiteit Gent, 9000 Gent (BE); The United States of America, as represented by The Secretary, Department of Health and Human Services, Bethesda, MD 20852-7660 (US); Trustees of Dartmouth College, Hanover, NH 03755 (US)
(72) Inventor: SAELENS, Xavier, 8900 Ieper (BE); SCHEPENS, Bert, 9031 Drongen (BE); ROSSEY, Iebe, 9050 Gentbrugge (BE); McLELLAN, Jason, Norwich, VT 05055 (US); GRAHAM, Barney, Rockville, MD 20850 (US)
(74) Representative: Iserentant, Hannes

(57) **Abstract**

The present invention relates to single domain antibodies that are directed against respiratory syncytial virus (RSV). More specifically, it relates to single domain antibodies that bind to the prefusion form of the fusion (F) protein of RSV. The invention relates further to the use of these single domain antibodies for prevention and/or treatment of RSV infections, and to pharmaceutical compositions comprising these single domain antibodies.

## Description

### Field of the invention

The present invention relates to single domain antibodies that are directed against respiratory syncytial virus (RSV). More specifically, it relates to single domain antibodies that bind to the prefusion form of the fusion (F) protein of RSV. The invention relates further to the use of these single domain antibodies for prevention and/or treatment of RSV infections, and to pharmaceutical compositions comprising these single domain antibodies.

### Background

Respiratory syncytial virus is the most important cause of acute airway infections in infants and young children. By the age of two nearly all children will have undergone at least one RSV infection. Although usually causing only mild disease, in a fraction of patients (1-2%) RSV infection leads to serious bronchiolitis where hospitalization is required. It has been estimated that each year 160.000 children die due to RSV infection. No effective prophylactic vaccine and no RSV-specific therapeutic small molecule are clinically developed. The only way in which high-risk infants can be partially protected from a severe disease caused by an anticipated RSV infection is by monthly injections with a humanized mouse monoclonal antibody directed against the pre- and postfusion conformation of the F protein of RSV (palivizumab). Nevertheless, treatment with this antibody is expensive and only used in a prophylactic setting. Several other antibodies are being developed, including a single domain antibody that binds to the F protein of RSV (WO2009147248). However, the monovalent nanobody has weak neutralization activity against RSV serotype B and multivalent formatting is needed to render the nanobody potent.

Recently, it was shown that conventional antibodies that specifically bind to the prefusion conformation of the RSV F protein are much more potent in vitro RSV neutralizers than antibodies that bind both the post- and the prefusion conformation of F (WO2008147196, US2012070446, McLellan et al., 2013). However, conventional antibodies can be cumbersome to produce and their stability may be limited. Furthermore, due to their relatively large size conventional antibodies can be hindered in their cognate epitope recognition in complex samples or when other antibodies and ligands are occupying sites in the vicinity of their epitopes.

Accordingly, and as there is no widely accepted treatment available, there is an unmet need for a potent anti-RSV drug which can be used for effective treatment and/or prevention of RSV infections.

### Summary

It is surprisingly shown herein that monovalent single domain antibodies can have strong neutralization activity against both RSV serotypes (A and B). This is unexpected, as literature suggests that multivalent constructs are needed for potent inhibition of both serotypes of RSV. Accordingly, it is an object of the invention to provide single domain antibodies directed against epitopes of the RSV F protein that are unique to the prefusion conformation and thereby providing highly potent single domain antibodies for the treatment and and/or prevention of RSV infections.

It is an object of the invention to provide a single domain antibody that binds specifically to the prefusion form of the F protein of RSV.

According to particular embodiments, the single domain antibody shows in monovalent format a similar neutralization activity of RSV serotypes A and B. Typically, this means that the single domain antibody inhibits infection of serotype A RSV virus and of serotype B RSV virus.

According to particular embodiments, the single domain antibody comprises a CDR1 sequence selected from the group consisting of SEQ ID N° 1 and SEQ ID N° 2, a CDR2 sequence selected from the group consisting of SEQ ID N° 3 and SEQ ID N° 4 and a CDR3 sequence selected from the group consisting of SEQ ID N° 5 and SEQ ID N° 6.

According to particular embodiments, a RSV binding construct comprising at least one single domain antibody against the RSV F protein is provided. At least one single domain antibody means that the RSV binding construct may contain more than one single domain antibody. Next to the one or more single domain antibodies, the RSV binding construct may contain other moieties linked to the single domain antibody. Said further moieties may bind to RSV or not.

According to particular embodiments, the single domain antibodies are not provided as such, but are provided as nucleic acids, i.e. nucleic acid molecules encoding single domain antibodies against the RSV F protein as herein described, particularly against the prefusion form of the F protein. According to yet further embodiments, host cells are provided containing such nucleic acids. Typically, such host cells will have been transformed or transfected with the nucleic acids. A particular use that is envisaged for these host cells is the production of the single domain antibodies. Thus, host cells transformed or transfected with the nucleic acid molecules encoding single domain antibodies can be used for production of the single domain antibodies.

According to a further aspect, the single domain antibodies are provided herein for use in medicine. That is to say, the single domain antibodies against RSV F protein are provided for use as a medicament. The same goes for the nucleic acid molecules encoding the single domain antibodies, i.e. it is envisaged that nucleic acid molecules encoding the single domain antibodies are provided for use as a medicament. According to particular embodiments, the single domain antibodies (or nucleic acids encoding them) are provided for use in therapeutic treatment or prevention of RSV infections. Likewise, the RSV binding construct comprising at least one single domain antibody against the RSV F protein is provided for use as a medicament. Particularly, these constructs are provided for use in therapeutic treatment or prevention of RSV infections.

This is equivalent as saying that methods are provided for therapeutic treatment or prevention of RSV infections for a subject in need thereof, comprising administering a single domain antibody against RSV F protein, and particularly to the prefusion form of the F protein, to said subject. Such methods typically will result in improvement or prevention of symptoms of the infection in said subject.

Here also, the single domain antibody may be provided as protein (as single domain protein, as part of a RSV binding construct or pharmaceutical composition) or may be administered as a nucleic acid molecule encoding a single domain antibody against RSV F protein, or as a vector comprising such nucleic acid molecule. Also, RSV binding constructs as described herein are envisaged for administration to a subject in need thereof in methods for therapeutic treatment or prevention of RSV infections.

If the single domain antibody (or the RSV binding construct) is administered as protein, different routes of administration can be envisaged. As non-limiting examples, the single domain antibody may be administered systemically, orally or intranasally, such as e.g. through nasal inhalation. In case the single domain antibody is provided as a nucleic acid or vector, it is particularly envisaged that the single domain antibody is administered through gene therapy.

According to a further aspect, pharmaceutical compositions are provided comprising at least one single domain antibody directed to RSV F protein. Typically, such pharmaceutical compositions comprise at least one single domain antibody directed to the prefusion form of RSV F protein. Said pharmaceutical compositions may comprise further moieties. Said further moieties may bind to RSV or not. It is envisaged herein that the pharmaceutical compositions are provided for use as a medicament. Particularly, they are provided for use in therapeutic treatment or prevention of RSV infections. This is equivalent as stating that methods are provided for therapeutic treatment or prevention of RSV infections for a subject in need thereof, comprising administering a pharmaceutical composition as described herein to said subject.

### Brief description of the Figures

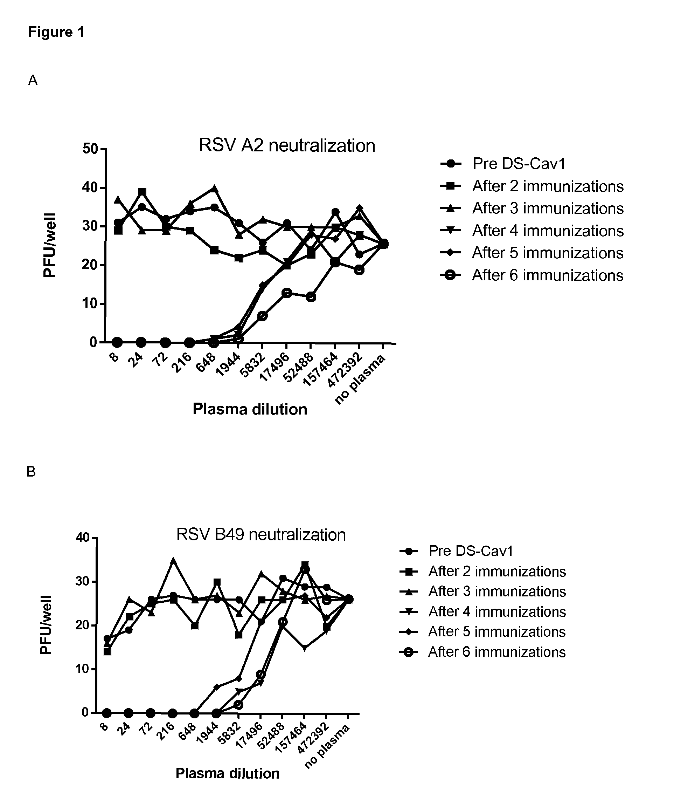

**Figure 1****: Neutralizing activity in plasma from llama immunized with DS-Cav1.** Neutralizing activity against RSV A2 (A) and RSV B49 (B) was tested in the plasma obtained after different immunizations with DS-Cav1. Monolayers of Vero cells seeded in 96-well plates were infected with RSV A2 or RSV B49 in the absence (no plasma) or presence of llama plasma, threefold diluted as indicated in the X-axis and starting with eightfold dilution. The number of plaques in each well was counted and is depicted in the Y-axis.

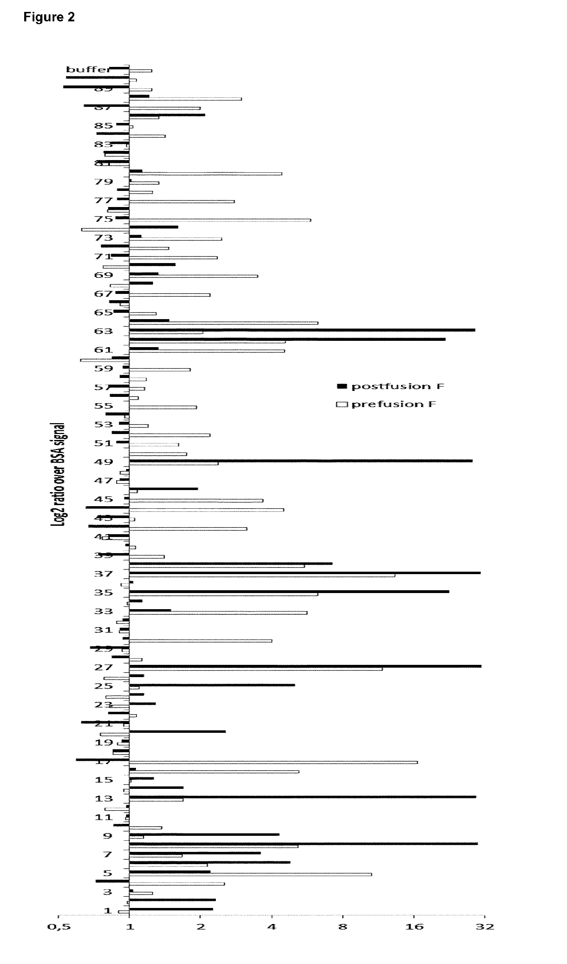

**Figure 2****: Selection of VHHs that bind to purified recombinant RSV fusion protein (F).** ELISA plates were coated with DS-Cav1 (blue bars, prefusion F), postfusion F (red bars) or BSA. The plates were incubated with bacterial periplasmic space extracts prepared from pHEN4-VHH transformed TG1 E. coli cells that had been obtained after one round of panning against DS-Cav1 with the VHH-displaying phage library generated from the PBMCs of the DS-Cav1 immunized llama. In the graph, the binding to the F protein is depicted as the log2 ratio of OD450 values over binding to BSA.

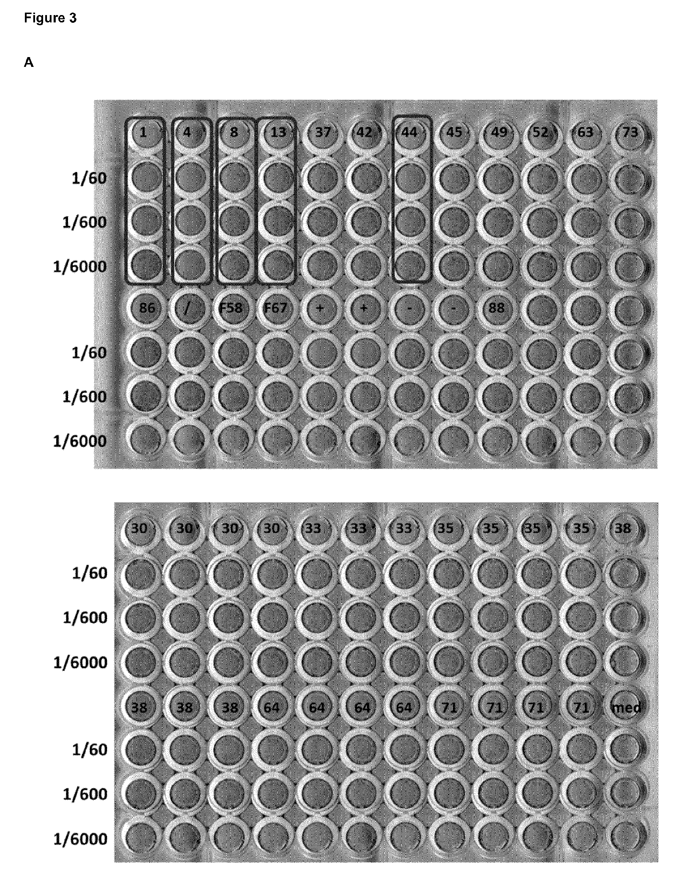

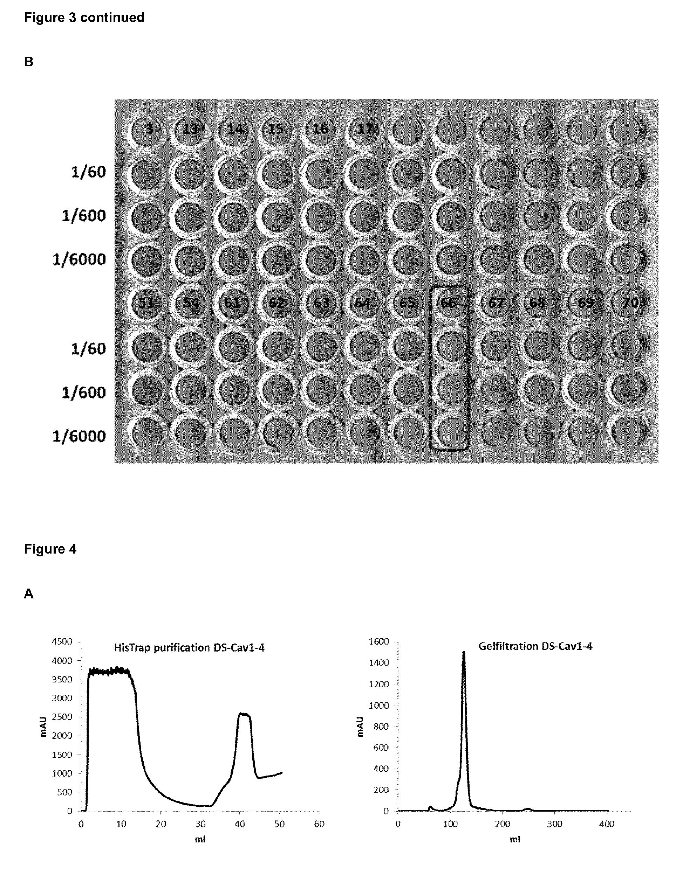

**Figure 3****: Detection of RSV neutralizing activity in Pichia pastoris supernatants.** Dilution series of Pichia pastoris supernatant of the individually picked Pichia clones (A) or the clones obtained after transformation of the complete VHH library (B) were mixed with a constant amount of RSV A2 and were put on Vero cells during 3 days after which a plaque assay was performed. The Figure shows the microtiter plates in which RSV plaques were stained with polyclonal anti-RSV serum. Wells incubated with supernatant from P. pastoris clones that appear to have neutralizing activity are indicated with a box.

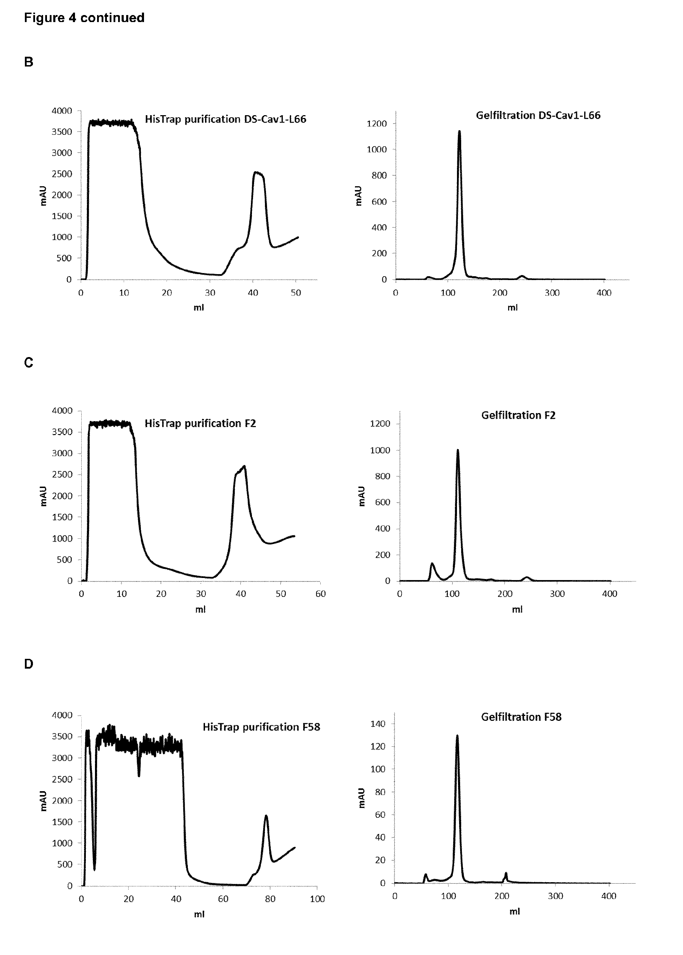

**Figure 4****: Purification of VHHs produced in Pichia pastoris.** The culture medium of P. pastoris cells that had been transformed with pKai-VHH-DS-Cav1-4, pKai-VHH-DS-Cav1-L66, pKai-VHH-F2 or pKai-VHH-F58 was harvested after induction with methanol for 96 h. Resolubilized ammonium sulphate precipitate of the cell-free medium was loaded onto a HisTrap column and after washing the column was eluted with an increasing concentration gradient of imidazole (left). The peak fraction that eluted from the HisTrap column was subsequently loaded on a Superdex 75 gelfiltration column (right). The chromatographs are shown for VHH-DS-Cav1-4 (A), VHH-DS-Cav1-L66 (B), VHH-F2 (C) and VHH-F58 (D).

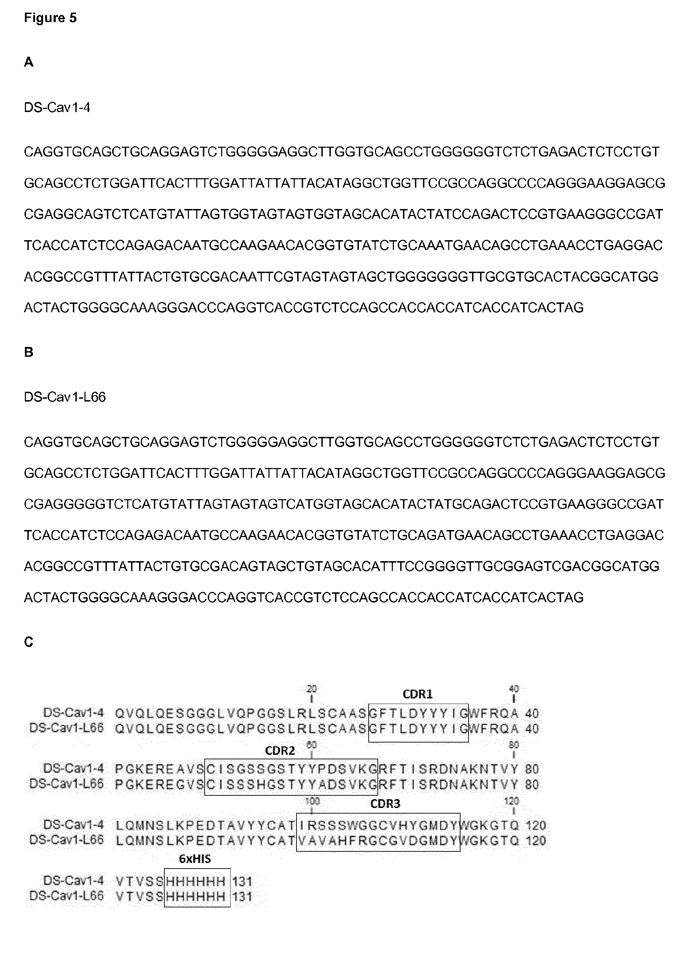

**Figure 5****:** Nucleotide sequences of VHH-DS-Cav1-4 (A) and VHH-DS-Cav1-L66 (B) and predicted amino acid sequences of recombinant VHH-DS-Cav1-4 and VHH-DS-Cav1-L66 produced in P. pastoris (C). The Complementarity Determining Regions (CDRs) and His tag (6xHIS) are indicated by labeled boxes.

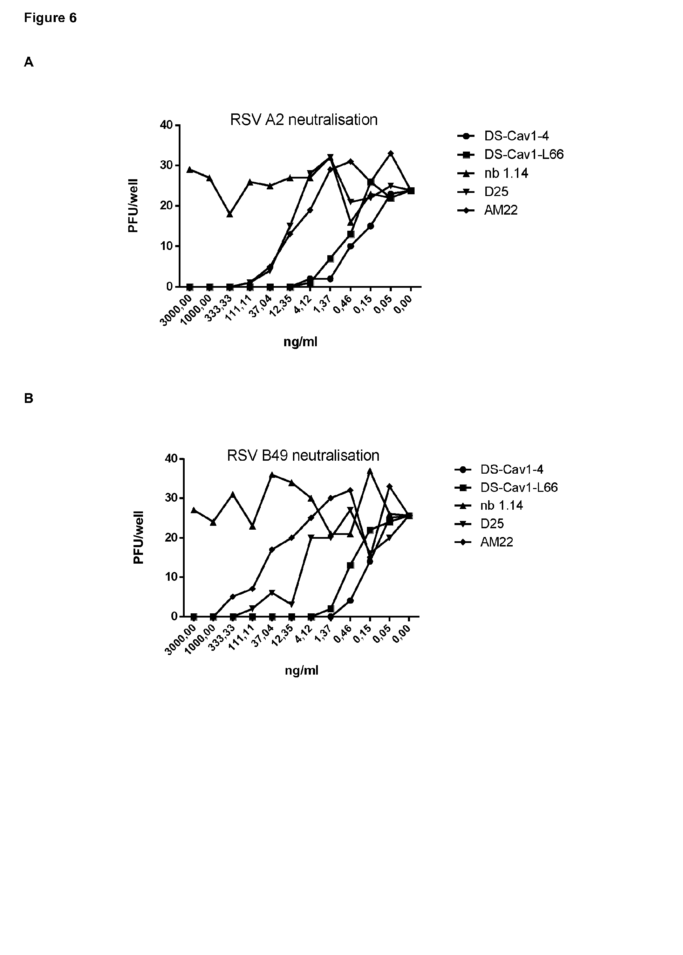

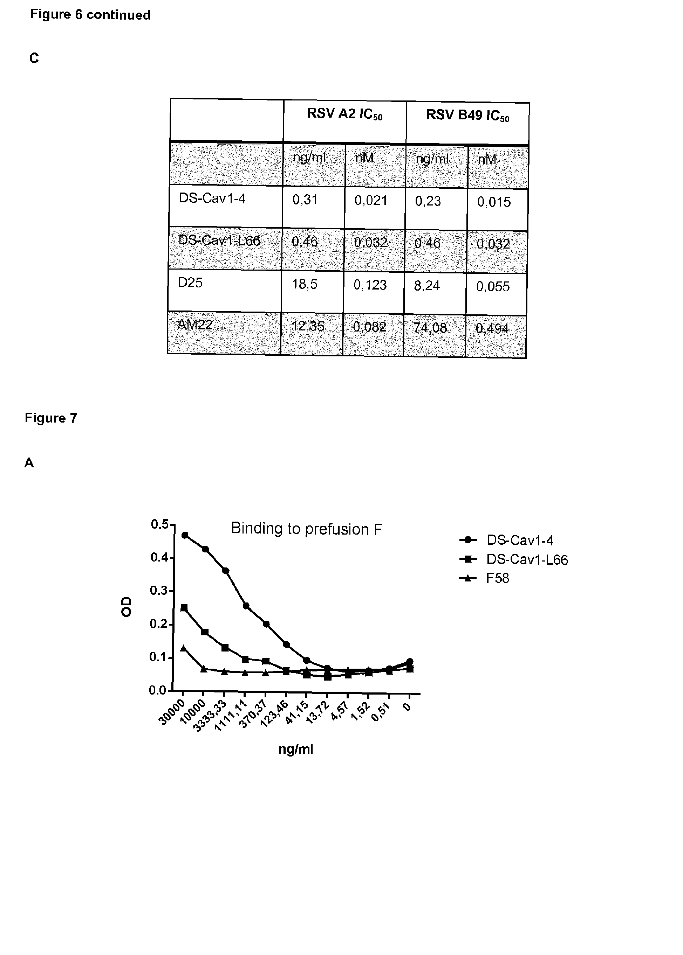

**Figure 6****: VHH-DS-Cav1-4 and VHH-DS-Cav1-L66 have potent RSV neutralizing activity.** Vero cells were infected with RSV A2 (A) or RSV B49 (B) in the presence of purified VHH (VHH-DS-Cav1-4, VHH-DS-Cav1-L66 or negative control NB 1.14), monoclonal antibody D25 or monoclonal antibody AM22. VHHs and monoclonal antibodies were applied in a threefold dilution series starting with a concentration of 3,000 ng/ml. The 50% inhibitory concentration (IC50) was calculated based on the plaque reduction shown in A and B and is depicted in (C).

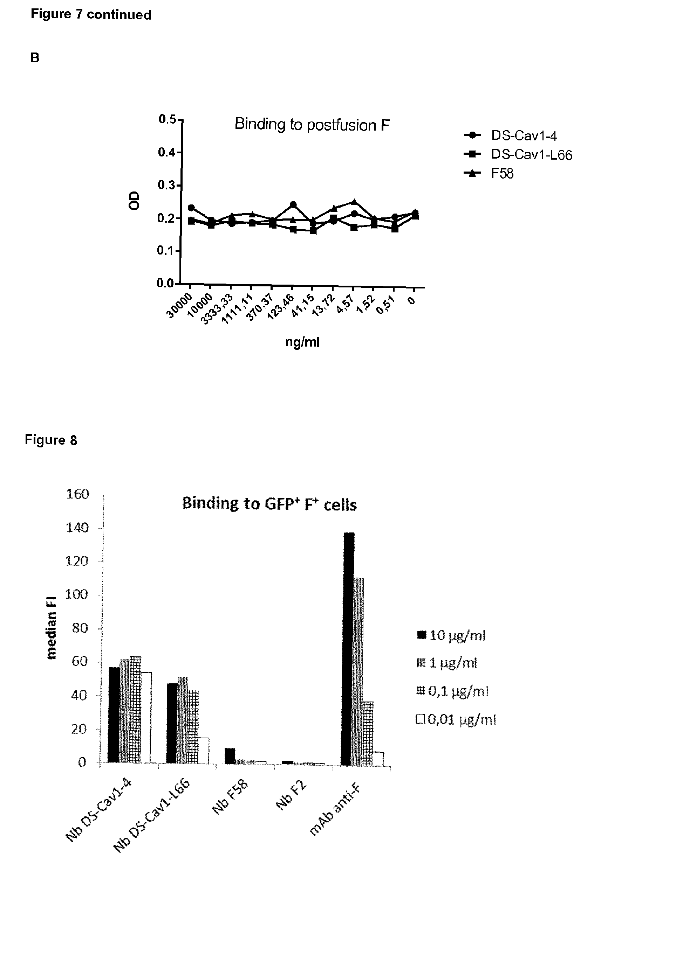

**Figure 7****: VHH-DS-Cav1-4 and VHH-DS-Cav1-L66 bind to DS-Cav1 but not to postfusion F.** ELISA plates were coated with DS-Cav1 (A) and postfusion F (B). The plates were incubated with a 1/3 dilution series of purified VHH-DS-Cav1-4 (purple line), VHH-DS-Cav1-L66 (blue line) and VHH-F58 (orange line) starting from 30,000 ng/µl. The OD450 values are depicted.

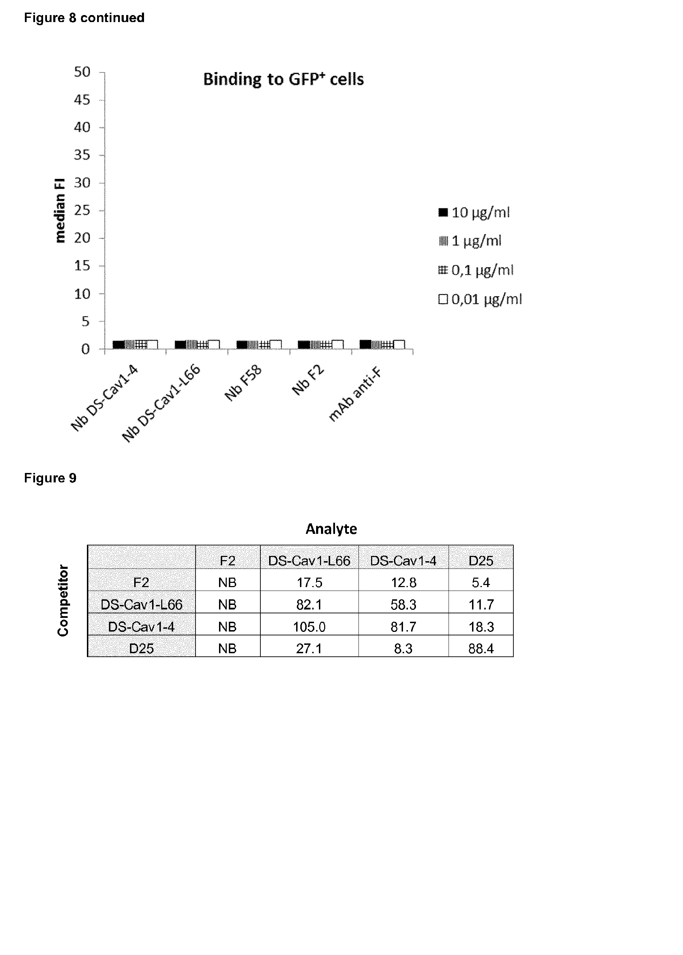

**Figure 8****: VHH-DS-Cav1-4 and VHH-DS-Cav1-L66 bind to F on the surface of mammalian cells.** HEK-293T cells were transfected with a RSV A2 F protein expression vector (pCAGGS-Fsyn) in combination with a GFP-NLS expression vector (peGFP-NLS) or transfected with only the GFP-NLS expression vector.
The graph shows the median fluorescence intensity (FI) of the indicated VHHs and an RSV F specific mouse monoclonal antibody to GFP positive cells expressing either the RSV F protein (top graph) or not (bottom graph).
**Figure 9****: Antibody cross-competition on DS-Cav1-binding analyzed by using biolayer interferometry.** DS-Cav1 was immobilized on AR2G biosensors through amine coupling reaction in acetate buffer. The reaction was quenched by 1M ethanolamine and DS-Cav1-immobilized biosensors were then equilibrated with assay buffer (PBS with 1% BSA). The biosensors were dipped in competitor antibodies/VHHs followed by analyte antibodies/VHHs with a short baseline step in between two antibody/VHH steps. Percent inhibitions were defined by comparing binding maxima of the analyte antibody/VHH in the absence and presence of each competitor. NB: not binding.

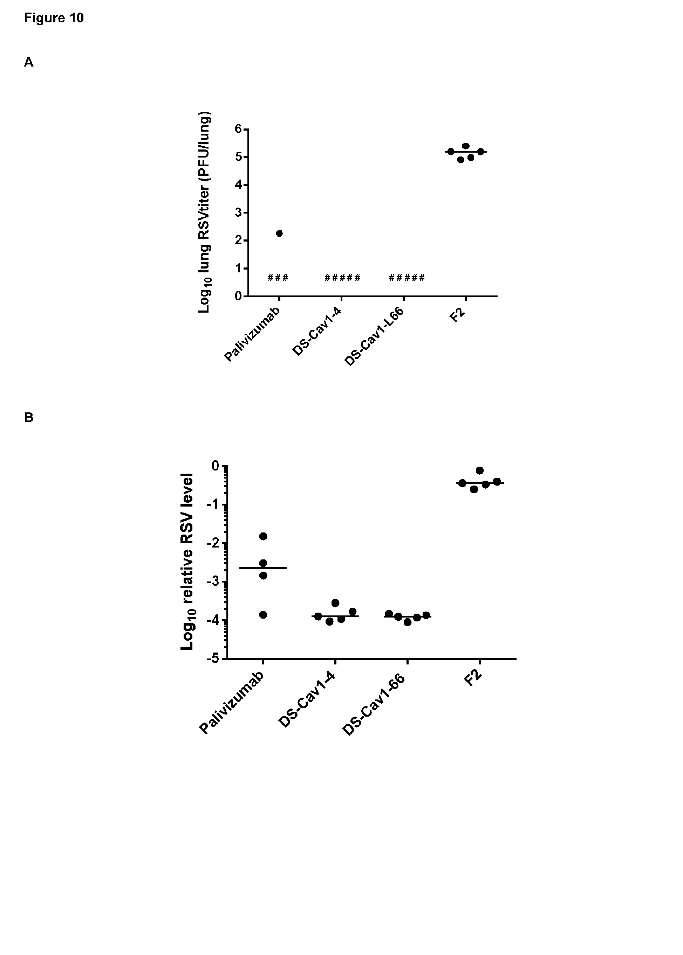

**Figure 10****: Prophylactic administration of VHH-DS-Cav1-4 and VHH-DS-Cav1-L66 reduces RSV replication in vivo.** 30 µg of VHH-DS-Cav1-4, VHH-DS-Cav1-L66 or F2, and 30 µg of palivizumab was administered intranasally to BALB/c mice four h prior to challenge with RSV A2. Twenty four h after infection all mice received 30 µg of F2 intranasally. Mice were sacrificed on day five after challenge and the virus load in the lungs was determined by plaque assay (A) and by qRT-PCR (B). Each data point represents one mouse and the horizontal lines depict the median. #: Mouse with virus titer in lung homogenate below detection limit. Graph (B) represents the relative expression of RSV RNA, normalized to mRPL13A mRNA levels present in the samples of each mouse in the indicated groups.

### Detailed description

### Definitions

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The following terms or definitions are provided solely to aid in the understanding of the invention. Unless specifically defined herein, all terms used herein have the same meaning as they would to one skilled in the art of the present invention. Practitioners are particularly directed to Sambrook et al., Molecular Cloning: A Laboratory Manual, 2nd ed., Cold Spring Harbor Press, Plainsview, New York (1989); and Ausubel et al., Current Protocols in Molecular Biology (Supplement 47), John Wiley & Sons, New York (1999), for definitions and terms of the art. The definitions provided herein should not be construed to have a scope less than understood by a person of ordinary skill in the art.

A "single domain antibody" is an antibody fragment consisting of a single variable antibody domain. Like a whole antibody, it is able to bind selectively to a specific antigen. With a molecular weight of only 12-18 kDa, single domain antibodies are much smaller than conventional antibodies (150-160 kDa) which are composed of two heavy and two light protein chains, and even smaller than Fab fragments (∼50 kDa, one light chain and half a heavy chain) and single chain variable fragments (∼25 kDa, two variable domains, one from a light and one from a heavy chain). The term "single domain antibody" as used herein includes variable domains of camelid heavy chain antibodies (VHHs), also referred to as Nanobodies, domain antibodies (dAbs), and single domain antibodies derived from shark (IgNAR domains).

The "prefusion form of the F protein of RSV" refers to the metastable prefusion conformation of the RSV F protein, that is adopted before virus-cell interaction, as described in McLellan et al., 2013. It is distinct from the highly stable postfusion form or postfusion conformation, which is adopted upon fusion of the viral and cellular membranes.

The "monovalent format" of an antibody as used herein refers to an antibody format that can only recognize one antigenic determinant. It excludes multivalent antibody formats that can recognize more than one antigenic determinant, such as - but not limited to - bivalent, trivalent or tetravalent formats.

The term "neutralization activity" as used herein, refers to the fact that the single domain antibody can inhibit virus infection as measured in an in vitro virus neutralization assay, such as - but not limited to - a plaque reduction assay. Neutralization, also referred to as inhibition, can mean full neutralization (no virus infection is observable) or may mean partial neutralization. For instance, neutralization can mean 10% neutralization, 20% neutralization, 25% neutralization, 30% neutralization, 40% neutralization or more. Particularly, neutralization will be at least 50%, e.g. 50% neutralization, 60% neutralization, 70% neutralization, 75% neutralization, 80% neutralization, 90% neutralization, 95% neutralization or more. The neutralization activity typically will be evaluated against a suitable control (e.g. treatment with an irrelevant single domain antibody), as will be readily chosen by the skilled person. For single domain antibodies with known concentration, the neutralization activity can be expressed as 50% inhibitory concentration (IC50). The IC50 is the single domain antibody concentration at which 50% inhibition (or neutralization) is achieved. It is a measure of the inhibitory potential, also referred to as potency, of a single domain antibody.

A "similar neutralization activity" as used herein, refers to a neutralization activity, expressed as IC50, that typically differs ten-fold or less from the neutralization activity it is compared to. More particularly, it differs five-fold or less, or even two-fold or less.

"Therapeutic treatment of a RSV infection", as used herein, means any form of treatment of a RSV infection that is administered to a subject after said subject contracted a RSV infection.

"Prevention of a RSV infection", as used herein, means a prophylactic treatment of a RSV infection that is administered to a subject before said subject contracted a RSV infection. Prophylactic treatment may include the use of the present invention as a vaccine. If this is the case, then typically, the present invention will be used as vaccine for passive immunization.

A "pharmaceutical composition", as used herein, may be any pharmaceutical composition known to the person skilled in the art, including, but not limited to compositions for systemic, oral and intranasal delivery.

A "RSV binding construct that comprises at least one single domain antibody", as used herein, refers to any binding construct that binds to RSV and comprises one or more single domain antibodies.

A "host cell", as used herein, may be any cell that is suitable for production of single domain antibodies or RSV binding constructs.

According to a first aspect, it is an objective of the invention to provide a single domain antibody that binds specifically to the prefusion form of the fusion (F) protein of respiratory syncytial virus (RSV). Typically, binding to the F protein of RSV means that the single domain antibody binds to the wild type form of the F protein as well as to any mutant form of the F protein, as long as the F protein is in the prefusion conformation. It also includes binding to the F protein of both subtypes (A and B) of RSV. According to particular embodiments, the single domain antibody shows in monovalent format a similar neutralization activity of RSV serotypes A and B. Typically, that means that the single domain antibody interferes with/inhibits/prevents/reverses or slows the ability of the virus to infect a cell. According to these particular embodiments, the single domain antibody interferes with/inhibits/prevents/reverses or slows the ability of the virus to infect a cell to a similar extent for both the A and B RSV serotypes.

According to particular embodiments, the single domain antibody comprises a CDR1 sequence selected from the group consisting of SEQ ID N° 1 and SEQ ID N° 2, a CDR2 sequence selected from the group consisting of SEQ ID N° 3 and SEQ ID N° 4 and a CDR3 sequence selected from the group consisting of SEQ ID N° 5 and SEQ ID N° 6. For the single domain antibody VHH-DS-Cav1-4, the sequences of the CDRs are SEQ ID N° 1, SEQ ID N° 3 and SEQ ID N° 5. For the single domain antibody VHH-DS-Cav1-L66 the sequences of the CDRs are SEQ ID N° 2, SEQ ID N° 4 and SEQ ID N° 6.

The single domain antibody may be provided as such or may be fused to further moieties.

According to a further aspect, a RSV binding construct is provided, characterized in that said RSV binding construct comprises at least one single domain antibody. Typically, said RSV binding construct binds to the RSV F protein. More specifically, said RSV binding construct binds to the prefusion form of the RSV F protein. Said RSV binding construct may be any construct comprising one or more than one RSV binding single domain antibody. Furthermore, said RSV binding construct may comprise further moieties. Said further moieties may bind to RSV or not. As a non-limiting example, said RSV binding construct may comprise a single domain antibody that binds to RSV F protein and may be linked (chemically or otherwise) to one or more groups or moieties that extend the half-life (such as - but not limited to - PEG), so as to provide a derivative of an single domain antibody of the invention with increased half-life.

According to a further aspect, the single domain antibodies are not provided as such, but are provided as nucleic acid, i.e. nucleic acid molecules encoding single domain antibodies against the RSV F protein as herein described. Also provided are vectors comprising such nucleic acids or nucleic acid molecules. According to yet a further aspect, host cells are provided comprising such nucleic acids or such vectors. Typically, the nucleic acids will have been introduced in the host cell by transfection or transformation; although the way in which the nucleic acid is introduced in the host cell is not limiting the invention. The host cells comprising the nucleic acids (or vectors) described herein are particularly suited for production of the single domain antibodies. Thus, such use for production is explicitly envisaged herein.

According to a further aspect, the single domain antibodies provided herein are for use in medicine. That is to say, the single domain antibodies against RSV F protein are provided for use as a medicament. The same goes for the nucleic acid encoding the single domain antibodies, or for the vectors containing such nucleic acids. Also the RSV binding constructs comprising at least one single domain antibody that binds to RSV F protein are provided for use as a medicament. According to particular embodiments, the single domain antibodies (or RSV binding constructs comprising them or pharmaceutical compositions comprising them or nucleic acids encoding them, or vectors comprising such nucleic acids) are provided for use in treatment or prevention of a RSV infection. This is equivalent as saying that methods are provided for treatment or prevention of a RSV infection for a subject in need thereof, comprising administering a single domain antibody against RSV F protein to said subject. Here also, the single domain antibody may be provided as protein (as single domain protein, as part of a RSV binding construct or pharmaceutical composition) or may be administered as a nucleic acid molecule encoding a single domain antibody against RSV F protein, or as a vector comprising such nucleic acid molecule. If the single domain antibody is administered as protein, different routes of administration can be envisaged. As non-limiting examples, the single domain antibody may be administered systemically, orally or intranasally, such as e.g. through nasal inhalation. In case the single domain antibody is provided as a nucleic acid or vector, it is particularly envisaged that the single domain antibody is administered through gene therapy.

According to a further aspect, pharmaceutical compositions are provided comprising at least one single domain antibody directed to RSV F protein. Typically, such pharmaceutical compositions comprise at least one single domain antibody directed to the prefusion form of RSV F protein. Said pharmaceutical compositions may comprise further moieties. Said further moieties may bind to RSV or not.

According to further embodiments, methods are provided for therapeutic treatment or prevention of RSV infections for a subject in need thereof, comprising administering a single domain antibody against RSV F protein, and particularly to the prefusion form of the F protein, to said subject. Such methods typically will result in improvement or prevention of symptoms of the infection in said subject.

Here also, the single domain antibody may be provided as protein (as single domain protein, as part of a RSV binding construct or pharmaceutical composition) or may be administered as a nucleic acid molecule encoding a single domain antibody against RSV F protein, or as a vector comprising such nucleic acid molecule, or as a pharmaceutical composition containing such antibody. Also, RSV binding constructs as described herein are envisaged for administration to a subject in need thereof in methods for therapeutic treatment or prevention of RSV infections.

It is to be understood that although particular embodiments, specific configurations as well as materials and/or molecules, have been discussed herein for cells and methods according to the present invention, various changes or modifications in form and detail may be made without departing from the scope and spirit of this invention. The following examples are provided to better illustrate particular embodiments, and they should not be considered limiting the application. The application is limited only by the claims.

### Examples

### Materials and methods to the examples

### Immunization and VHH library generation

A llama was injected subcutaneously on days 0, 7, 14, 21, 28 and 35, each time with 167 µg of purified RSV F protein DS-Cav1. DS-Cav1 is a recombinant RSV F protein stabilized in the prefusion conformation (McLellan et al., 2013). The first two injections were performed with poly-IC (375 µg per injection) as adjuvant, while Gerbu LQ # 3000 was used as adjuvant for the last four injections. Before every immunization blood was taken and plasma prepared to evaluate seroconversion. On day 40, 100 ml of anticoagulated blood was collected for the preparation of lymphocytes.

Total RNA from peripheral blood lymphocytes was used as template for first strand cDNA synthesis with oligodT primer. Using this cDNA, the VHH encoding sequences were amplified by PCR, digested with PstI and NotI, and cloned into the PstI and NotI sites of the phagemid vector pHEN4. Electro-competent E. coli TG1 cells were transformed with the recombinant pHEN4 vector resulting in a VHH library of about 5 x 108 independent transformants. 87% of the transformants harbored the vector with the right insert size, as evidenced by PCR analysis of 95 independent transformants.

500 µl of the library stock was infected with VCS M13 helper phage in order to display the VHH sequences (in fusion with M13 PIII) on the phage surface, which were used for bio-panning.

### Cells

Hep-2 cells (ATCC, CCL-23), Vero cells (ATCC, CCL-81) and HEK-293T cells (a gift from Dr M. Hall) were grown in DMEM medium supplemented with 10% heat-inactivated fetal calf serum (FCS), 2mM L-glutamine, non-essential amino acids (Invitrogen, Carlsbad, California) and 1 mM sodium pyruvate.

### Viruses

RSV A2 (VR-1540, ATCC, Rockville), an A subtype of RSV, and RSV B49, a B subtype of RSV (BE/5649/08 clinical strain, obtained from Prof M. Van Ranst, Tan et al., 2013) were propagated by infecting monolayers of Hep-2 cells, with 0.1 MOI in the presence of growth medium containing 1% FCS. Five days after infection the cells and growth medium were collected, pooled and clarified by centrifugation (450 x g). To concentrate the virus, the clarified supernatant was incubated for four h at 4°C in the presence of 10% polyethylene glycol (PEG6000). After centrifugation (30 minutes at 3000 x g), the pellet was resuspended in Hank's balanced salt solution (HBSS), containing 20% sucrose, aliquoted, snap-frozen in liquid nitrogen and stored at -80°C.

### RSV-neutralizing activity assay

The llama plasma was tested for neutralizing activity against RSV A2 and RSV B49 by plaque assay. Vero cells were seeded in a 96-well plate (15000 cells/well). The next day, a dilution series of the plasma samples was prepared in Opti-MEM (Gibco) supplemented with 1% penicillin and 1% streptomycin (1/3 dilution series, starting with a 1/4 dilution). An equal volume of RSV A2 suspension (diluted to 1.4 PFU/µl) or RSV B49 (diluted to 2.8 PFU/µl) was added to the plasma samples and the obtained mixtures were incubated for 30 minutes at 37°C. Subsequently, 50 µl of the mixtures was added to the Vero cells, which had been washed with Opti-MEM, and the cells were incubated at 37°C for three h. Next, 50 µl of 1.2 % avicel in DMEM medium supplemented with 2% heat-inactivated FCS, 2mM L-glutamine, non-essential amino acids and 1 mM sodium pyruvate was added to each well and the infection was allowed to continue at 37°C for three days. The cells were fixed for 30 minutes at room temperature by adding 50 µl of a 2% paraformaldehyde solution to the wells. After fixation, the cells were washed twice with phosphate-buffered saline (PBS), permeabilized with 50 µl PBS with 0.2% Triton X-100 for 10 minutes and blocked with PBS containing 1% BSA. Subsequently, polyclonal goat anti RSV serum (AB1125, Chemicon International) was added (1/2000 in PBS containing 0.5% BSA and 0.001% Triton X-100 (PBS/BSA)). After three washes with PBS/BSA the cells were incubated with horseradish peroxidase-conjugated anti-goat IgG (SC2020, Santa Cruz) for 30 minutes. The wells were subsequently washed four times with PBS/BSA and once with PBS. Finally, the plaques were visualized by applying TrueBlue peroxidase substrate (KPL, Gaithersburg). RSV neutralizing activity in crude Pichia pastoris supernatant and of purified VHHs (see below) was also determined with this assay.

### Isolation of DS-Cav1 binding VHHs

We performed one round of panning to enrich for prefusion F (DS-Cav1) binding phages. One well (well A1) on a microtiter plate (type II, F96 Maxisorp, Nunc) was coated overnight with 20 µg DS-Cav1 in PBS. This well, along with an uncoated, negative control well (well A12) was blocked with SEA BLOCK blocking buffer (Thermo Scientific) for one h. Next, 1012 phages in a volume of 100 µl SEA BLOCK blocking buffer were added to these two wells. After one h, the unbound phage particles were removed and the wells were washed ten times with PBST (PBS + 0.5% Tween20). The retained phages were then eluted by applying an alkaline solution consisting of 100 µl of TEA-solution (14% triethylamine (Sigma) pH 10) to the wells for exactly ten minutes. The dissociated phages were then transferred to a sterile tube with 100 µl 1M TRIS-HCl pH 8.0. Tenfold serial dilutions in PBS were prepared with the eluted phages, and 10 µl of this dilution series was used to infect 90 µl of TG1 cells (phage display competent E. coli cells). Infection was allowed for 30 minutes at 37°C, after which the bacteria were plated on LB/agar plates with 100 µg/ml ampicillin and 1% glucose. The enrichment for antigen-specific phages by this panning procedure was assessed by comparing the number of phagemid particles eluted from antigen-coated well with the number of phagemid particles eluted from the negative control well.

Ninety ampicillin resistant colonies were randomly selected for further analysis by ELISA for the presence of F-specific VHHs in their periplasm. These colonies were first transferred to a fresh LB agar plate with ampicillin and then used to inoculate 1 ml of Terrific Broth (TB) medium with 100 µg/ml ampicillin in a 24 deep well plate. Inoculated plates were incubated at 37°C while shaking for five h. VHH expression was induced by adding isopropyl β-D-1-thiogalactopyranoside (IPTG) until a concentration of 1 mM. The plates were subsequently incubated overnight at 37°C while shaking. The next day, bacterial cells were pelleted by centrifugation (12 minutes at 3200 rpm) and the supernatant was removed. The cell pellet was resuspended in 200 µl TES buffer (0.2 M TRIS-HCl pH 8, 0.5 mM EDTA, 0.5 M sucrose) and the plates were shaken at 4°C for 30 minutes. Next, water was added to the resuspended cells to induce an osmotic shock, which leads to the release of the periplasmic proteins that include VHHs. The deep well plates were incubated for one h at 4°C while shaking, centrifuged and the supernatant, containing the periplasmic extract was recovered. Four microtiter plates were coated overnight with 100 ng of protein per well in PBS, two with alternating rows of F in the postfusion conformation (McLellan et al., 2011) and BSA, two others with alternating rows of DS-Cav1 and BSA. The coated microtiter plates were then washed and blocked with 1% milk powder in PBS. After washing of the microtiter plates, 100 µl of periplasmic extract was added to the wells and followed by incubation for one h at 4°C. The plates were washed and 50 µl of a 1/2000 dilution of anti-HA (MMS-101P Biolegend) monoclonal antibody in PBS was added to the plate for one h at room temperature. After washing, a 1/2000 dilution in PBS of horseradish peroxidase (HRP)-linked anti-mouse IgG (NXA931, GE Healthcare) was added and the plates were incubated during one hour. Next, the plates were washed and 50 µl of TMB substrate (Tetramethylbenzidine, BD OptEIA™) was added to every well. The reaction was stopped by addition of 50 µl of 1M H2SO4 after which the absorbance at 450 nM was measured with an iMark Microplate Absorbance Reader (Bio Rad). All periplasmic fractions for which the OD450 values obtained for DS-Cav1 or postfusion F were at least two times higher than the OD450 values obtained for BSA, were selected for further analysis. The corresponding bacteria were grown in 3 ml of LB medium with 1/2000 ampicillin for plasmid isolation using the QIAprep Spin Miniprep kit (Qiagen) The cDNA sequence of the cloned VHH was determined by Sanger sequencing using M13RS primer (5'CAGGAAACAGCTATGACC3').

### Cloning of VHH into Pichia pastoris expression vector and transformation of Pichia pastoris

The VHH sequences, as well as the VHH sequences that were retained after the panning, were PCR amplified from the respective pHEN4 plasmids using the following forward and reverse primers (5'GGCGGGTATCTCTCGAGAAAAGGCAGGTGCAGCTGCAGGAGTCTGGG3'; 5'CTAACTAGTCTAGTGATGGTGATGGTGGTGGCTGGAGACGGTGACCTGG3'). The resulting PCR products were digested with Xhol and Spel and ligated into Xhol/Spel digested pKai61 backbone. The origin of the pKai61 vector is described in Schoonooghe et al., 2009. The VHH sequences are cloned in frame with a slightly modified version of the S. cerevisiae a-mating factor signal sequence. This signal sequence directs the proteins to the yeast secretory system, is further processed in the ER and the golgi and will be fully removed before secretion into the extracellular medium. In contrast to the wild-type prepro signal, this modified version does not contain sequences that code for the GluAla repeats (here the signal peptide is efficiently cleaved by the Kex2 endopeptidase without the need for this repeat). The encoded genes contain a C-terminal 6xHis tag and are under control of the methanol inducible AOX1 promoter. The plasmid contains a Zeocine resistance marker for selection in bacterial as well as in yeast cells. The vectors were linearized in the AOX1 promoter (with Pmel) before transformation to P. pastoris to promote homologous recombination in the endogenous AOX1 locus for stable integration into the genome. The resulting vectors were named pKai-VHH-DS-Cav1-4, pKai-VHH-DS-Cav1-L66, pKai-VHH-F2 and pKai-VHH-F58 and used to transform Pichia pastoris strain GS115 using the condensed transformation protocol described by Lin-Cereghino et al., 2005.

### Purification of VHHs produced by Pichia pastoris

Expression of VHH by transformed Pichia pastoris clones was first analysed in 2ml cultures. On day one individual transformants were used to inoculate 2 ml of YPNG medium (2% pepton, 1% Bacto yeast extract, 1.34% YNB, 0.1M potassium phosphate pH6, 0.00004% biotine, 1% glycerol) with 100 µg/ml Zeocin (Life Technologies) and incubated while shaking at 28°C for 24 h. The next day, cells were pelleted by centrifugation (8 minutes at 500 g) and the YPNG medium was replaced by YPNM medium (2% pepton, 1% Bacto yeast extract, 1.34% YNB, 0.1M potassium phosphate pH6, 0.00004% biotine, 1% methanol) to induce VHH expression and cultures were incubated at 28°c while shaking for 72 h. Fifty 50 µl of 50% methanol was added to the cultures at 72 h, 80 h and 96 h. One hundred h after transfer to methanol containing medium the yeast cells were pelleted by centrifugation (8 minutes at 500 g) and the supernatant was retained to assess the presence of VHH. Crude medium (25 µl) was loaded on a 15% SDS-PAGE gel, after which presence of protein was analysed by Coomassie Brilliant Blue staining. To select VHHs with RSV neutralizing activity, we determined such activity in the crude YPNM supernatant from individual Pichia pastoris transformants by applying serial dilutions of the supernatant in a plaque assay as described above.

Pichia pastoris transformants that yielded high levels of VHH in the medium or with high RSV neutralizing activity were selected for scale up using 100 or 300ml Pichia cultures. Growth and methanol induction conditions, and harvesting of medium were similar as mentioned above for the 2 ml cultures. The cleared medium was subjected to ammonium sulphate precipitation (80% saturation) for four hours at 4°C. The insoluble fraction was pelleted by centrifugation at 20,000 g and solubilized in 10 ml HisTrap binding buffer (20 mM sodium phosphate, 0.5 M NaCl, 20 mM imidazole, pH 7.4), centrifuged for 10 minutes at 4°C after which the supernatant was loaded on a 1 ml HisTrap HP column (GE Healthcare), pre-equilibrated with the HisTrap binding buffer. After washing the column with at least 10 column volumes of HisTrap binding buffer (until the absorbance reaches a steady baseline), the bound proteins were eluted with a linear imidazole gradient starting from 20 mM and ending at 500 mM imidazole in HisTrap binding buffer over a total volume of 20 ml. Fractions containing the VHH, as determined by SDS-PAGE analysis were pooled, and concentrated to 2 ml with a Vivaspin column (5kDa cutoff, GE Healthcare). These concentrated fractions were then loaded on a Superdex 75 column (160 ml, 0.8 ml/min) in PBS and peak fractions were pooled and concentrated on a Vivaspin column with a 5kDa cutoff. The protein concentration of the pooled fraction was determined by A280 measurement by NanoDrop 1000 with the percent solution extinction coefficient customized to each VHH. The pooled and concentrated fractions were aliquoted and stored at -80°C before further use.

### Calculation of 50% inhibitory concentration (IC50) of purified VHHs

To determine the IC50 of the purified VHHs produced by Pichia pastoris threefold serial dilutions prepared in Opti-Mem of these VHHs were evaluated in an RSV neutralization assay as described above. Monoclonal IgGs D25 and AM22 (Beaumont et al., 2012, Spits et al., 2013), both specifically directed at the prefusion conformation of F, were used as positive controls. NB 1.12, a VHH directed against α-macroglobulin was used as a negative control. IC50 values were calculated manually.

### In vitro binding of VHHs to DS-Cav1 and postfusion F

The binding of the purified VHHs to DS-Cav1 and postfusion F was tested in a direct ELISA. Microtiter plates (type II, F96 Maxisorp, Nunc) were coated with 100 µl of a 1 µg/ml DS-Cav1 solution or a 1 µg/ml postfusion F solution in PBS. After washing, the plates were blocked for one h with 200 µl of 4% milk in PBS after which they were washed again with PBS once. A 1/3 dilution series of the VHHs (starting from 30 µg/ml) was then applied to the protein-coated wells. After one hour, the plates were washed and a 1/2000 dilution of anti-Histidine Tag antibody (AD1.1.10 AbD Serotec) in PBS was added for an hour. After washing and addition of HRP-linked anti-mouse IgG during one h (in a 1/2000 dilution), the ELISA was developed in the same way as the PE-ELISA described above.

Binding of the VHHs to F expressed on the surface of cells that had been transfected with an RSV F cDNA expression vector was evaluated by flow cytometry. HEK293T cells were seeded at 4,000,000 cells per 150 mm tissue culture plate and transfected with 6.4 µg pCAGGS-Fsyn, which encodes a codon-optimized RSV F cDNA, with the FuGENE HD transfection reagent (Promega). To trace transfected cells, transfections were performed in the presence of 6.4 µg of peGFP-NLS. Control transfections were performed with peGFP-NLS only. Eighteen h after transfection the cells were detached with 15 ml trypsin-EDTA solution (0.05% trypsin, 0.5 mM EDTA (pH 8.0)) washed once in PBS and incubated for 30 minutes in PBS containing 1% BSA (PBS/BSA). Subsequently the cells were incubated with the indicated VHH or with RSV-F specific mouse monoclonal antibody (MAB858-1, Chemicon International) at different concentrations as indicated in Figure 8). One h later the cells were washed once with PBS/BSA and incubated with anti-Histidine Tag antibody diluted 1/3000 in PBS/BSA during one h. Next, the cells were washed once with PBS/BSA and anti-mouse IgG Alexa 633 was added during 30 minutes. After washing the cells three times with PBS, the cells were analyzed using a FACSCalibur flow cytometer. Single GFP expressing cells were selected based on the peak surface of the sideward scatter signal, the peak surface and peak height of the forward scatter signal and the peak surface of the green fluorescence signal. Finally, of these GFP positive single cells, the Alexa 633 fluorescence intensity signal was measured.

### Mice

Specific pathogen-free, female BALB/c mice were obtained from Charles River (Charles River Wiga, Sulzfeld, Germany). The animals were housed in a temperature-controlled environment with 12 h light/dark cycles; food and water were provided ad libitum. The animal facility operates under the Flemish Government License Number LA1400536. All experiments were done under conditions specified by law and authorized by the Institutional Ethical Committee on Experimental Animals (Ethical application EC2015-019).

### Administration of VHHs and monoclonal antibodies and RSV challenge of mice

Mice were slightly anesthesized by isoflurane before intranasal administration of VHH, palivizumab or RSV challenge virus. VHH, palivizumab (Synagis, Medimmune) and RSV virus were administered in a total volume of 50 µl formulated in PBS, which was distributed equally over the two nostrils. Each group of five mice received 30 µg of VHH DS-Cav1-4, 30 µg of VHH DS-Cav1-L66, 30 µg of VHH-F2 (as a negative control) or 30 µg of palivizumab (as a positive control) four hours before infection with 1,000,000 PFU of RSV A2. All groups also received 30 µg of the negative control VHH-F2 24h after infection.

### Determination of lung viral titers by plaque assay

Five days after challenge, the mice were sacrificed by cervical dislocation. The mouse lungs were removed aseptically and homogenized by vigorous shaking with a Mixer Mill MM 2000 (Retsch) in the presence of a sterile metal bead in 1 ml HBSS containing 20% sucrose and supplemented with 1% penicillin and 1% streptomycin. Lung homogenates were subsequently cleared by centrifugation (10 minutes at 2500 rpm) at 4°C and used in duplicate for virus titration on Vero cells. Monolayers of Vero cells were infected with 50 µl of serial 1:3 dilutions of lung homogenates in a 96-well plate in serum-free Opti-MEM medium (Invitrogen) supplemented with penicillin and streptomycin. The plaque assay was further processed as described above. The plaques in each well were counted and for each dilution the number of PFU per lung (1ml) was calculated as follows: number of plaques present in the dilution x the dilution x 20 (= 1000 µl total supernatant volume / 50 µl of supernatant used to infect the first well of the dilution series). The number of PFU in each lung was than calculated as the average of the duplicates.

### Determination of lung viral titer by qRT-PCR

To determine the lung RSV load by qRT-PCR, total RNA from the cleared lung homogenates was prepared by using the High Pure RNA tissue Kit (Roche, Mannheim) according to the manufacturer's instructions. cDNA was prepared by the use of random hexamer primers and the Transcriptor First strand cDNA synthesis kit (Roche, Mannheim). The relative levels of genomic RSV M cDNA were determined by qRT-PCR using primers specific for the RSV A2 M gene (5'TCACGAAGGCTCCACATACA3' and 5'GCAGGGTCATCGTCTTTTTC3') and a nucleotide probe (#150 Universal Probe Library, Roche) labeled with fluorescein (FAM) at the 5'-end and with a dark quencher dye near the 3'- end. The qRT-PCR data were normalized to mRPL13A mRNA levels present in the samples of each mouse.

### Antibody cross-competition on DS-Cav1-binding

DS-Cav1 protein (10 µg/ml) was immobilized on AR2G biosensors through amine coupling reaction in acetate buffer (pH 5.0). The reaction was quenched by 1M ethanolamine and DS-Cav1-immobilized biosensors were then equilibrated with assay buffer (PBS with 1% BSA). The biosensors were dipped in competitor antibodies (35 µg/ml in assay buffer) followed by analyte antibodies (35 µg/ml in assay buffer) with a short baseline step in between two antibody steps.

### Example 1: RSV neutralizing activity in llama plasma

To assess the induction of humoral anti-RSV responses in the llama after immunization with DS-Cav1, plasma samples obtained before and after each immunization were tested in a RSV-neutralization assay. The samples were tested for their neutralizing activity against RSV-A2 and a clinical strain of RSV B, RSV B49. Figure 1 illustrates that all plasma samples obtained after the fourth immunization have high neutralizing activity against RSV A2 and RSV B49.

### Example 2: Isolation of DS-Cav1-specific VHHs

The VHH phage library was subjected to one round of panning on the DS-Cav1 protein. The enrichment for DS-Cav1-specific phages was assessed by comparing the number of phages eluted from DS-Cav1-coated wells with the number of phages eluted from the uncoated wells. The number of eluted phages was estimated indirectly by determining the ampicillin resistance transducing units, i.e. the number of TG1 colonies that had been transduced with the phages eluted in the panning step. This experiment suggested that the phage population was enriched about 140-fold for DS-Cav1-specific phages. Ninety colonies were randomly selected and analyzed by ELISA for the presence of VHHs specific for the prefusion conformation of F (DS-Cav1) versus the postfusion conformation of F in their periplasmic extracts. The result of this ELISA is depicted in Figure 2. Out of the 90 colonies, 37 colonies scored positive (10 scored positive for binding to both pre- and postfusion F, 19 scored positive for binding to only prefusion F and 8 scored positive for binding to only postfusion F). The VHH sequence of all colonies that suggested binding to pre- and or postfusion F was determined. Twenty eight clones out of 37 had a unique VHH sequence and were selected for further use. The VHH sequences of these clones were cloned into a Pichia pastoris expression vector and the resulting plasmids were subsequently used to transform Pichia pastoris.

### Example 3: Testing neutralizing activity in Pichia pastoris supernatants

We also attempted to clone the VHH cDNA library obtained after one round of panning on DS-Cav1 into the Pichia pastoris expression vector pKai61. We used this strategy in order to try to select biologically relevant VHH candidates based on RSV neutralizing activity in the supernatant of individual Picha pastoris transformants. From the 20 individual Pichia pastoris transformants selected based on binding to F, five had neutralizing activity against RSV A2 (with VHH DS-Cav1-4 being the most potent one), while from 18 clones obtained from the library cloning into pKai61, only one (VHH DS-Cav1-L66) displayed neutralizing activity (Figure 3).

The cDNA sequence of VHH DS-Cav1-4 and DS-Cav1-L66 was determined by Sanger sequencing and the nucleotide sequence as well as the deduced amino acid sequence are shown in Figure 5.

### Example 4: Production and determination of IC50 of purified VHHs

Prior to purification, VHH DS-Cav1-4 and VHH DS-Cav1-L66 had the most potent RSV neutralizing VHHs and these two VHHs as well as negative control VHHs F2 and F58 were produced in 300 ml Pichia pastoris cultures and purified by HisTrap purification followed by superdex 75 size exclusion chromatography (Figure 4). VHH F2 and VHH F58 are irrelevant control VHHs obtained from a VHH library derived from a different llama that had been immunized with inactived Junin virus. VHH DS-Cav1-4 and VHH DS-Cav1-L66 in vitro neutralized RSV A2 with an IC50 of 0.021 nM and 0.032 nM, respectively. For neutralization of RSV B49 VHH DS-Cav1-4 and VHH DS-Cav1-L66 displayed an IC50 of 0.015 nM and 0.032 nM, respectively.

### Example 5: Testing binding to fusion protein

To evaluate the binding affinity of VHH DS-Cav1-4 and VHH DS-Cav1-L66 to pre- and postfusion F, we performed an ELISA in which F in either conformation was coated directly on the microtiter plate and a threefold dilution series of VHHs was added to this plate (Figure 7). We found that VHH DS-Cav1-4 and VHH DS-Cav1-L66 bound specifically to coated prefusion F and not to coated F in the postfusion conformation (Figure 7, compare A and B).

Binding of VHH DS-Cav1-4 and VHH DS-Cav1-L66 to F expressed by mammalian cells was also evaluated. HEK293T cells were co-transfected with pCAGGS-Fsyn, encoding a codon optimized F cDNA and peGFP-NLS. Cells were harvested 18 h after transfection and stained with VHH DS-Cav1-4, VHH DS-Cav1-L66, negative control VHHs F58 and F2 and a monoclonal mouse IgG antibody specific for RSV-F. Binding of the VHHs to the GFP positive cells in the co-transfection setting was compared with binding to GFP positive cells that had been transfected with the GFP expression vector only. Clear binding was observed for all dilutions of VHH DS-Cav1-4 and VHH DS-Cav1-L66 and for the three highest concentrations of the positive control monoclonal antibody directed against RSV F (Figure 8).

### Example 6: VHH-DS-Cav1-4 and VHH-DS-Cav1-L66 do not compete with the epitope Ø specific antibody D25 for the binding to the RSV prefusion F protein.

To investigate whether VHH-DS-Cav1-4 and VHH-DS-Cav1-L66 bind to the recently described prefusion F specific epitope Ø, we investigated if these VHHs can compete with the epitope Ø specific D25 antibody for the binding to the RSV prefusion F protein by biolayer interferometry (Figure 9). The competition assay illustrates that neither VHH competes with D25. In contrast, both VHHs did compete with each other. These results indicate that both VHHs bind to overlapping epitopes, but those epitopes are different from the D25 epitope Ø.

### Example 7: Testing prophylactic anti-RSV activity of VHHs in vivo

To test if prophylactic administration of VHH DS-Cav1-4 or DS-Cav1-L66 can protect against RSV challenge in vivo, female BALB/c mice (five mice per group) received 30 µg of VHH DS-Cav1-4, VHH DS-Cav1-L66, F2 VHH or palivizumab intranasaly four h before infection with 1.106 PFU of RSV A2). Twenty four h after challenge, all mice received 30 µg of F2 VHH intranasally. Five days after challenge, the mice were sacrificed and lungs were homogenized in 1 ml of HBSS supplemented with 20% sucrose, penicillin and streptomycin. Mice that had been treated with DS-Cav1-4, DS-Cav1-L66 or palivizumab had no detectable replicating virus in their lungs (except one mouse treated with palivizumab) (Figure 10 A) in contrast to the group which had been treated with the F2 VHH which displayed high levels of replicating virus in their lungs (about 1x105 PFU). As plaque assays used to quantify the level of replicating virus in the lungs can be affected by the presence of neutralizing antibodies or VHHs, we additionally quantified the level of RSV RNA in the lung homogenates by qRT-PCR. Mice that had been treated with VHH-DS-Cav1-4 and VHH-DS-Cav1-L66 displayed on average more than 3000 times less viral RNA as compared to the F2 treated control mice. Mice that had been treated with palivizumab displayed on average about 100 fold less viral RNA as compared to the F2 treated control mice.

### References

1. Lin-Cereghino, J., et al Condensed protocol for competent cell preparation and transformation of the methylotrophic yeast Pichia pastoris. Biotechniques, 38, 44, 46, 48 (2005).
2. McLellan, J.S., et al. Structure of respiratory syncytial virus fusion glycoprotein in the postfusion conformation reveals preservation of neutralizing epitopes. J Virol, 85, 7788-96 (2011).
3. McLellan, J.S., et al. Structure of RSV fusion glycoprotein trimer bound to a prefusion-specific neutralizing antibody. Science 340, 1113-1117 (2013).
4. McLellan, J.S., et al. Structure-based design of a fusion glycoprotein vaccine for respiratory syncytial virus. Science 342, 592-598 (2013).
5. Schoonooghe, S., et al. Efficient production of human bivalent and trivalent anti-MUC1 Fab-scFv antibodies in Pichia pastoris. BMC Biotechnol., 9, 70 (2009).
6. Tan, L., et al. The comparative genomics of human respiratory syncytial virus subgroups A and B: genetic variability and molecular evolutionary dynamics. J Virol. 87, 8213-26 (2013).

## Claims

1. A single domain antibody, **characterized in that** said single domain antibody binds specifically to the prefusion form of the fusion (F) protein of respiratory syncytial virus (RSV).

2. The single domain antibody according to claim 1, **characterized in that** said single domain antibody shows in monovalent format a similar neutralization activity of RSV serotypes A and B.

3. The single domain antibody according to claim 1 or 2, **characterized in that** said single domain antibody comprises a CDR1 sequence selected from the group consisting of SEQ ID N° 1 and SEQ ID N° 2, a CDR2 sequence selected from the group consisting of SEQ ID N° 3 and SEQ ID N° 4 and a CDR3 sequence selected from the group consisting of SEQ ID N° 5 and SEQ ID N° 6.

4. A RSV binding construct, **characterized in that** said RSV binding construct comprises at least one single domain antibody according to anyone of claims 1 to 3.

5. A nucleic acid, **characterized in that** said nucleic acid encodes at least one single domain antibody according to anyone of claims 1 to 3.

6. A host cell, **characterized in that** said host cell is transformed or transfected with the nucleic acid according to claim 5.

7. The use of the host cell according to claim 6, for the production of said single domain antibody.

8. The single domain antibody according to anyone of claims 1 to 3 or the RSV binding construct according to claim 4, for use as a medicament.

9. The single domain antibody according to anyone of claims 1 to 3 or the RSV binding construct according to claim 4, for use in therapeutic treatment or prevention of a RSV infection.

10. A pharmaceutical composition, **characterized in that** said pharmaceutical composition comprises at least one single domain antibody according to anyone of claims 1 to 3.

11. The pharmaceutical composition according to claim 10, for use as a medicament.

12. The pharmaceutical composition according to claim 10, for use in therapeutic treatment or prevention of a RSV infection.

13. A method of therapeutic treatment or prevention of a RSV infection, the method comprising administering to a subject in need thereof the single domain antibody according to anyone of claims 1 to 3, the RSV binding construct according to claim 4 or the pharmaceutical composition according to claim 10.


SEQUENCE LISTING

<110>  VIB VZW
       UNIVERSITEIT GENT
       THE UNITED STATES OF AMERICA, as represented by the
       Secretary, Department of Health and Human Services, National
       Institutes of Health, Office of Technology Transfer
       TRUSTEES OF DARTMOUTH COLLEGE

<120>  SINGLE DOMAIN ANTIBODY AGAINST RSV F PROTEIN

<130>  XaSa/RSVNb/521

<160>  15

<170>  PatentIn version 3.5

<210>  1
<211>  10
<212>  PRT
<213>  Artificial Sequence

<220>
<223>  CDR1 sequence

<400>  1

Gly Phe Thr Leu Asp Tyr Tyr Tyr Ile Gly
1               5                   10


<210>  2
<211>  10
<212>  PRT
<213>  Artificial Sequence

<220>
<223>  CDR1 sequence

<400>  2

Gly Phe Thr Leu Asp Tyr Tyr Tyr Ile Gly
1               5                   10


<210>  3
<211>  17
<212>  PRT
<213>  Artificial Sequence

<220>
<223>  CDR2 sequence

<400>  3

Cys Ile Ser Gly Ser Ser Gly Ser Thr Tyr Tyr Pro Asp Ser Val Lys
1               5                   10                  15


Gly

<210>  4
<211>  17
<212>  PRT
<213>  Artificial Sequence

<220>
<223>  CDR2 sequence

<400>  4

Cys Ile Ser Ser Ser His Gly Ser Thr Tyr Tyr Ala Asp Ser Val Lys
1               5                   10                  15

Gly

<210>  5
<211>  16
<212>  PRT
<213>  Artificial Sequence

<220>
<223>  CDR3 sequence

<400>  5

Ile Arg Ser Ser Ser Trp Gly Gly Cys Val His Tyr Gly Met Asp Tyr
1               5                   10                  15

<210>  6
<211>  16
<212>  PRT
<213>  Artificial Sequence

<220>
<223>  CDR3 sequence

<400>  6

Val Ala Val Ala His Phe Arg Gly Cys Gly Val Asp Gly Met Asp Tyr
1               5                   10                  15

<210>  7
<211>  396
<212>  DNA
<213>  Artificial Sequence

<220>
<223>  single domain antibody DS-Cav1-4

<220>
<221>  CDS
<222>  (1)..(396)

<400>  7
cag gtg cag ctg cag gag tct ggg gga ggc ttg gtg cag cct ggg ggg        48
Gln Val Gln Leu Gln Glu Ser Gly Gly Gly Leu Val Gln Pro Gly Gly
1               5                   10                  15

tct ctg aga ctc tcc tgt gca gcc tct gga ttc act ttg gat tat tat          96
Ser Leu Arg Leu Ser Cys Ala Ala Ser Gly Phe Thr Leu Asp Tyr Tyr
            20                  25                  30

tac ata ggc tgg ttc cgc cag gcc cca ggg aag gag cgc gag gca gtc          144
Tyr Ile Gly Trp Phe Arg Gln Ala Pro Gly Lys Glu Arg Glu Ala Val
        35                  40                  45

tca tgt att agt ggt agt agt ggt agc aca tac tat cca gac tcc gtg          192
Ser Cys Ile Ser Gly Ser Ser Gly Ser Thr Tyr Tyr Pro Asp Ser Val
    50                  55                  60

aag ggc cga ttc acc atc tcc aga gac aat gcc aag aac acg gtg tat          240
Lys Gly Arg Phe Thr Ile Ser Arg Asp Asn Ala Lys Asn Thr Val Tyr
65                  70                  75                  80

ctg caa atg aac agc ctg aaa cct gag gac acg gcc gtt tat tac tgt          288
Leu Gln Met Asn Ser Leu Lys Pro Glu Asp Thr Ala Val Tyr Tyr Cys
                85                  90                  95

gcg aca att cgt agt agt agc tgg ggg ggt tgc gtg cac tac ggc atg          336
Ala Thr Ile Arg Ser Ser Ser Trp Gly Gly Cys Val His Tyr Gly Met
            100                 105                 110

gac tac tgg ggc aaa ggg acc cag gtc acc gtc tcc agc cac cac cat          384
Asp Tyr Trp Gly Lys Gly Thr Gln Val Thr Val Ser Ser His His His
        115                 120                 125

cac cat cac tag                                                          396
His His His
    130


<210>  8
<211>  131
<212>  PRT
<213>  Artificial Sequence

<220>
<223>  Synthetic Construct

<400>  8

Gln Val Gln Leu Gln Glu Ser Gly Gly Gly Leu Val Gln Pro Gly Gly
1               5                   10                  15


Ser Leu Arg Leu Ser Cys Ala Ala Ser Gly Phe Thr Leu Asp Tyr Tyr
            20                  25                  30


Tyr Ile Gly Trp Phe Arg Gln Ala Pro Gly Lys Glu Arg Glu Ala Val
        35                  40                  45


Ser Cys Ile Ser Gly Ser Ser Gly Ser Thr Tyr Tyr Pro Asp Ser Val
    50                  55                  60


Lys Gly Arg Phe Thr Ile Ser Arg Asp Asn Ala Lys Asn Thr Val Tyr
65                  70                  75                  80

Leu Gln Met Asn Ser Leu Lys Pro Glu Asp Thr Ala Val Tyr Tyr Cys
                85                  90                  95

Ala Thr Ile Arg Ser Ser Ser Trp Gly Gly Cys Val His Tyr Gly Met
            100                 105                 110

Asp Tyr Trp Gly Lys Gly Thr Gln Val Thr Val Ser Ser His His His
        115                 120                 125

His His His
    130

<210>  9
<211>  396
<212>  DNA
<213>  Artificial Sequence

<220>
<223>  Single domain antibody DS-Cav1-L66

<220>
<221>  CDS
<222>  (1)..(396)

<400>  9
cag gtg cag ctg cag gag tct ggg gga ggc ttg gtg cag cct ggg ggg      48
Gln Val Gln Leu Gln Glu Ser Gly Gly Gly Leu Val Gln Pro Gly Gly
1               5                   10                  15

tct ctg aga ctc tcc tgt gca gcc tct gga ttc act ttg gat tat tat      96
Ser Leu Arg Leu Ser Cys Ala Ala Ser Gly Phe Thr Leu Asp Tyr Tyr
            20                  25                  30

tac ata ggc tgg ttc cgc cag gcc cca ggg aag gag cgc gag ggg gtc      144
Tyr Ile Gly Trp Phe Arg Gln Ala Pro Gly Lys Glu Arg Glu Gly Val
        35                  40                  45

tca tgt att agt agt agt cat ggt agc aca tac tat gca gac tcc gtg      192
Ser Cys Ile Ser Ser Ser His Gly Ser Thr Tyr Tyr Ala Asp Ser Val
    50                  55                  60

aag ggc cga ttc acc atc tcc aga gac aat gcc aag aac acg gtg tat      240
Lys Gly Arg Phe Thr Ile Ser Arg Asp Asn Ala Lys Asn Thr Val Tyr
65                  70                  75                  80

ctg cag atg aac agc ctg aaa cct gag gac acg gcc gtt tat tac tgt      288
Leu Gln Met Asn Ser Leu Lys Pro Glu Asp Thr Ala Val Tyr Tyr Cys
                85                  90                  95

gcg aca gta gct gta gca cat ttc cgg ggt tgc gga gtc gac ggc atg      336
Ala Thr Val Ala Val Ala His Phe Arg Gly Cys Gly Val Asp Gly Met
            100                 105                 110

gac tac tgg ggc aaa ggg acc cag gtc acc gtc tcc agc cac cac cat      384
Asp Tyr Trp Gly Lys Gly Thr Gln Val Thr Val Ser Ser His His His
        115                 120                 125

cac cat cac tag                                                                 396
His His His
    130


<210>  10
<211>  131
<212>  PRT
<213>  Artificial Sequence

<220>
<223>  Synthetic Construct

<400>  10

Gln Val Gln Leu Gln Glu Ser Gly Gly Gly Leu Val Gln Pro Gly Gly
1               5                   10                  15


Ser Leu Arg Leu Ser Cys Ala Ala Ser Gly Phe Thr Leu Asp Tyr Tyr
            20                  25                  30


Tyr Ile Gly Trp Phe Arg Gln Ala Pro Gly Lys Glu Arg Glu Gly Val
        35                  40                  45


Ser Cys Ile Ser Ser Ser His Gly Ser Thr Tyr Tyr Ala Asp Ser Val
    50                  55                  60


Lys Gly Arg Phe Thr Ile Ser Arg Asp Asn Ala Lys Asn Thr Val Tyr
65                  70                  75                  80


Leu Gln Met Asn Ser Leu Lys Pro Glu Asp Thr Ala Val Tyr Tyr Cys
                85                  90                  95


Ala Thr Val Ala Val Ala His Phe Arg Gly Cys Gly Val Asp Gly Met
            100                 105                 110


Asp Tyr Trp Gly Lys Gly Thr Gln Val Thr Val Ser Ser His His His
        115                 120                 125


His His His
    130


<210>  11
<211>  18
<212>  DNA
<213>  Artificial Sequence

<220>
<223>  Primer

<400>  11
caggaaacag ctatgacc                                                              18

<210>  12
<211>  47
<212>  DNA
<213>  Artificial Sequence

<220>
<223>  Primer

<400>  12
ggcgggtatc tctcgagaaa aggcaggtgc agctgcagga gtctggg                    47


<210>  13
<211>  49
<212>  DNA
<213>  Artificial Sequence

<220>
<223>  Primer

<400>  13
ctaactagtc tagtgatggt gatggtggtg gctggagacg gtgacctgg                  49


<210>  14
<211>  20
<212>  DNA
<213>  Artificial Sequence

<220>
<223>  Primer

<400>  14
tcacgaaggc tccacataca                                                  20


<210>  15
<211>  20
<212>  DNA
<213>  Artificial Sequence

<220>
<223>  Primer

<400>  15
gcagggtcat cgtcttttc                                                   20